# EUROPEAN PATENT APPLICATION

(11) **EP 3 689 302 A1**
(43) Date of publication of application: **05.08.2020**
(21) Application number: 19382070.1
(22) Date of filing: 01.02.2019
(51) Int. Cl.: A61F 7/00, A61F 7/03, A61F 7/08, A61F 7/02

(54) **THERMAL MATTRESS FOR BODY SUPPORT**

(71) Applicant: Centre de Formacio I Docencia Formes S.L., 08410 Vilanova del Vallés (ES)
(72) Inventor: BRANDO GARRIDO, Cecilia, E-08410 Vilanova del Vallés (ES)
(74) Representative: Tribalyte Ideas

(57) **Abstract**

The present invention relates to, but is not limited to, a novel reusable thermal mattress designed for the thermal treatment of a patient, preferably used during the care and transportation of patients in emergency situations. To do so, said mattress (1) comprises a thermal transfer surface (2) with an outer face adapted to receive the body of a patient and an inner face, as well as a thermal compartment (3) arranged inside the mattress in contact with the inner face of the thermal transfer surface, the thermal compartment (3) being adapted to house a thermoactive substance. Lastly, it comprises transfer means (4) for transferring the thermoactive substance between the thermal compartment (3) and an area outside the mattress (1).

## Description

### FIELD OF THE INVENTION

The present invention falls within the technical field corresponding to devices adapted for thermal therapy or treatment on patients. More specifically, the invention relates to, but is not limited to, a mattress able to provide cold or heat to a patient based on the features of their condition, said mattress being suitable for being used in emergency situations when transporting the patient to a health centre.

### BACKGROUND OF THE INVENTION

There are certain health emergencies, such as severe inflammation in patients due to, for example, multiple traumas or cardiovascular diseases, or the rescue of hypothermic or hyperthermic patients at sea, in deserts or mountains, wherein a patient requires the early application of cold or heat treatments. In said situations, chemical and thermoelectric devices are generally used which require prior warming or cooling by means of electric systems connected to energy sources or single-use devices.

Thus, the electric solutions generically produce heat for hypothermic patients by means of blanket or mattress-type configurations. However, said electric devices require connection to the electrical grid or energy sources, where in the case of not having them, the device loses its thermal functionality. Furthermore, these energy sources are generally heavy and large, which hinders the portability of the invention in emergency situations.

In the case of single-use chemical devices, the use of bags that generically and initially contain two compartments separated by a joining area is known, wherein each compartment separately comprises a compound, one compartment has a chemical needed to generate an endothermic or exothermic chemical reaction, and the other contains water. Thus, upon breaking the joining area of the compartments and mixing both components, a chemical reaction is generated that produces a suitable amount of heat or cold to initially treat the patient. However, the aforementioned devices do not enable the device to be reused and the size thereof does not enable the entire body surface of the victim to be covered, in addition to being able to move during transport.

Likewise, other portable, folding and lightweight devices also exist, such as thermal survival blankets, but in this case the device only enables the temperature of the victim to be maintained at the time of rescue, the device itself not being able to generate energy to restore the minimal vital signs of the patient. As such, if the patient is in a state of hypothermia or hyperthermia, this device will not produce the opposite effect desired.

For example, the patent application US 2011190855 A1 discloses a cooling assembly comprising a support element and one or more cooling devices formed by an impermeable bag and a selectively breakable bag, which contain water and a reagent, respectively, such that when mixed they produce an endothermic reaction.

Patent EP 2468222 B1 discloses a device suitable for absorbing heat formed by a bag comprising two compartments hermetically separated from each other by means of a removable separation element. One of the compartments contains a solid composition and a substance that favours the formation of a gel, while the second compartment contains water and another gelling substance. Thus, upon removing the separation element, an endothermic reaction begins.

Patent US4282005A, on the contrary, discloses a device suitable for generating heat from an exothermic reaction that occurs between two components housed in the interior thereof. The reaction occurs when the components, initially separated, come in contact.

Furthermore, in many emergency situations, such as for example in the case of a patient with multiple traumas, it is of vital importance to immobilise them to prevent their state of health from worsening. To do so, devices such as vacuum mattresses are commonly used, which comprise a vacuum compartment that generally contains microballs of polyethylene and air. Thus, by positioning the patient on said mattress and removing the air from the inside of the vacuum compartment, the mattress takes the shape of the patient and acquires a rigid consistency. Therefore, the patient is immobilised and the vibrations during transportation to a medical centre are minimised. However, there is no product that enables the advantages of the vacuum mattresses and the advantages provided by the application of early thermal treatments to be simultaneously achieved by means of a single invention.

Therefore, the present invention has two objects, the first is to provide a thermal assembly for body support for a patient during emergency situations, capable of transferring both cold and hot thermal energy to the patient by means of a portable and reusable solution. Secondly, the present invention enables, in different preferred embodiments, the advantages provided by the vacuum mattresses to be combined with the advantages related to the early thermal treatment of the patient.

### BRIEF DESCRIPTION OF THE INVENTION

An object of the present invention relates to, but is not limited to, a thermal mattress for body support, suitable to be applied during the transportation of patients in emergency situations.

Advantageously, said device comprises:
a thermal transfer surface, with an outer face adapted to receive the body of a patient and an inner face;
a thermal compartment arranged inside the mattress in contact with the innerface of the thermal transfer surface, the thermal compartment being adapted to house a thermoactive substance;
transfer means for transferring the thermoactive substance between the thermal compartment and an area outside the mattress.

A portable, reusable and versatile thermal assembly is thus obtained, being able to contain thermoactive substances that generate or absorb heat as many times as necessary. Thus, the rescued victim can be treated the entire time they are transported to the hospital, and multiple patients in different emergency situations can be treated as any times as necessary, and early thermal treatments can be used, since they can be applied with heat or cold. The use of thermal substances makes it portable, and the transfer means make it reusable.

In this sense, inducing hyperthermia is especially suited for situations in which it is necessary to raise the patient's body temperature, for example, when said patient has suffered from hypothermia in situations involving emergency rescue at sea, in the mountains, in the snow or in isolation due to weather conditions. In turn, inducing hypothermia is advised for patients who have suffered ischaemic diseases, with damaged tissues, traumas or in rescues in desert areas.

Furthermore, it is important to specify that throughout the present document thermoactive substances are understood as any set of substances or chemical mixtures of reagents that generate exothermic or endothermic reactions, such as for example:
- chemical mixtures of reagents formed by a solution of magnesium sulphate and water;
- chemical mixtures of reagents formed by a supersaturated solution of sodium acetate trihydrate and water;
- chemical mixtures of reagents formed by ammonium in ratios greater than 95% with sodium carboxymethyl cellulose and with sodium chloride;
- chemical mixtures of nitrogenous salts and water;
- compounds with thermal inertia that can transfer sensible heat for a certain period of time, such as in the case of water; or
- compounds that can transfer latent heat.

In one embodiment of the invention, the thermal transfer surface is rough and slip resistant.

This prevents the patient from sliding on the mattress, but rather remain in the most appropriate position for receiving the thermal transfer.

In a preferred embodiment of the invention, the thermal compartment is distributed longitudinally through the inside of the mattress and is welded to the inner face of the thermal transfer surface.

This ensures contact between the thermal compartment and the thermal transfer surface. The thermal compartment can be tube-shaped, longitudinally passing through the length of the inner face of the thermal transfer surface. The gauge and length of the tube is proportionally adapted to house an amount of thermoactive substance, taking into account the changes in state that it will undergo; liquid-solid-liquid.

In a preferred embodiment of the invention, the mattress further comprises a vacuum compartment, the thermal compartment being located between said vacuum compartment and the thermal transfer surface, and wherein said vacuum compartment comprises:
a vacuum valve for injecting or removing a gas from the inside of the vacuum compartment;
a plurality of moulding granulated components inside the vacuum compartment.

The function of the vacuum compartment, in those embodiments where it is present, is generally intended for the transportation of patients who have suffered severe traumas and need to be immobilised. Thus, a device is obtained capable of achieving the en bloc mobilisation of the patient and absorbing most of the vibrations during the transportation of the patient to a medical centre. The thermal function enables a thermal treatment to be applied to said patient, providing or absorbing heat.

In particular embodiments, the thermal compartment and/or the vacuum compartment are impermeable to liquids and/or gases.

This sealing enables better functioning of both compartments.

In a preferred embodiment of the invention, the granulated components of the vacuum compartment are spheres that, in more preferred embodiments of the invention, comprise polyethylene.

These polyethylene spheres are cost-effective and carry out their function of providing consistency to the vacuum compartment, such that the protection of the patient during transportation to the medical centre is facilitated.

In a preferred embodiment of the invention, the material of the vacuum compartment comprises polyvinyl chloride or polyurethane.

In a preferred embodiment of the invention, the transfer means comprise at least two valves located in the thermal compartment.

Two valves have a reversible capacity for filling and emptying the thermal compartment.

In a preferred embodiment of the invention, the device further comprises temperature sensors arranged on the thermal transfer surface of the mattress.

These sensors enable the temperature to be measured in a way that the action of the thermoactive substance can be regulated.

In a preferred embodiment of the invention, the device additionally comprises one or more handles located on the sides of the mattress.

These handles allow the mattress to be transported in a simple way by the medical staff.

In a preferred embodiment of the invention, the device further comprises three or more straps.

These straps are suitable for securing the patient to the mattress when necessary.

In a preferred embodiment of the invention, each strap has an end joined in a sliding manner to a handle, such that by sliding the end along the handle, the position of the straps is adjusted.

This allows the straps to be placed at different points along the body of the patient. Since the handles are located on the sides of the mattress, when the handle is sufficiently long, it provides a path for the strap to be located in advantageous positions for the patient.

In a preferred embodiment of the invention, the thermal compartment comprises a mechanical actuator therein. More preferably, said actuator is a mechanical nucleation actuator, which may be necessary in some cases.

This achieves the possibility of using chemical substances that require an initiating event that disrupts the chemical balance of the thermoactive substance and initiates a chemical reaction, like in the case of using hypersaturated solutions of sodium acetate in water.

Another object of the present invention relates to a kit for the thermal treatment and body support of a patient comprising: a thermal assembly, according to any of the preferred embodiments described previously, a thermoactive substance and an element for injecting the thermoactive element inside the thermal compartment through the transfer means.

In a preferred embodiment of the invention, the injection means comprise a syringe with dimensions suitable for the transfer means, and wherein the kit additionally comprises a manual pump, an electric pump and/or a secretion aspirator suitable for filling or emptying the vacuum compartment.

### DESCRIPTION OF THE FIGURES

Figure 1 shows a perspective view of the thermal assembly for treating a patient, according to a preferred embodiment of the invention.
Figure 2 shows a side view of the thermal and vacuum assembly for treating a patient, according to a preferred embodiment of the invention, wherein a cross-section has been represented to show some of the internal elements of said mattress.

### NUMERICAL REFERENCES USED IN THE FIGURES

For a better understanding of the technical features of the invention, the aforementioned Figures are accompanied by a series of numerical references which, by way of illustration and not limitation, represent the following:

| | |
|---|---|
| (1) | Mattress |
| (2) | Thermal transfer surface |
| (3) | Thermal compartment |
| (4) | Valves of the thermal compartment |
| (5) | Vacuum compartment |
| (6) | Granulated components |
| (7) | Vacuum valve |
| (8) | Handles |
| (9) | Straps |

### DETAILED DESCRIPTION OF THE INVENTION

A preferred exemplary embodiment of the present invention, provided by way of illustration and not limitation of the same, is described below.

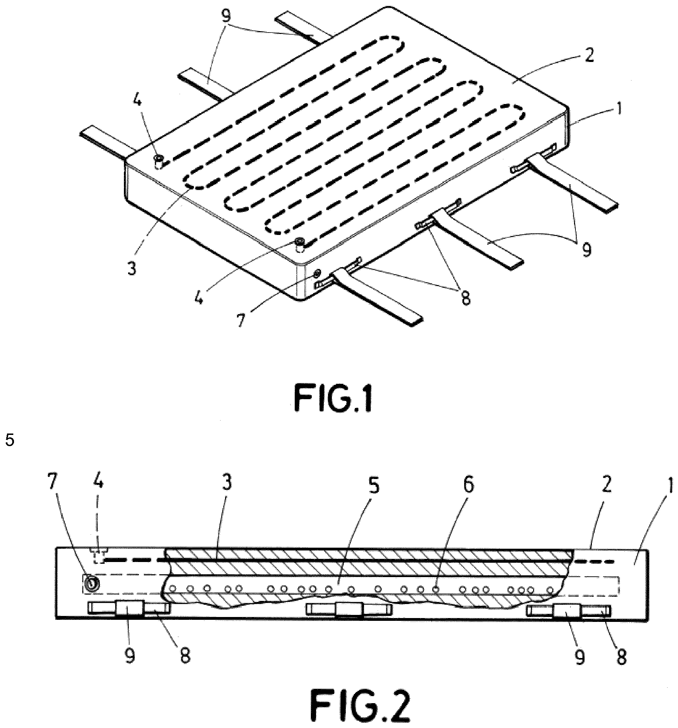

A main object of the invention relates to, as described in the preceding sections and as shown in Figures 1 and 2 of the present document, a thermal assembly for body support, preferably used during the care and transportation of patients in emergency situations to a medical centre. Said thermal assembly comprises, in a preferred embodiment, a mattress 1 with a thermal transfer surface 2 to the patient where the exchange of energy with the body of said patient is carried out. Based on the ailment or condition of the patient, thermal transfer can be for providing heat or for dissipating it. To generate said heat as well as said cold, a thermoactive substance capable of transferring cold or hot thermal energy is used for a specific amount of time until room temperature is reached once again. By way of example, said thermoactive substance could refer to a chemical mixture that generates an exothermic or endothermic reaction.

Thus, the mattress 1 of the invention comprises a thermal compartment 3 welded to the inner face of the surface of the mattress 1 arranged longitudinally and covering the width of the mattress, configured in a way that maintains thermal contact with the thermal transfer surface 2. Said thermal compartment 3 is impermeable and enables the thermoactive substance to be housed in the interior thereof, having the gauge and capacity needed to house the chemical substance and the changes in state thereof, going from liquid to solid and again to liquid, capable of generating both cold and heat to transfer to the patient through said thermal transfer surface 2. Likewise, the mattress 1 comprises means of transferring the thermoactive substance between the inside of the thermal compartment 3 and an area outside the mattress 1. Even more preferably, said transfer means for transferring the thermoactive substance are formed by two or more valves 4 joined to the longitudinal tube that forms the thermal compartment 3. Said valves are adapted to enable the thermoactive substance to be introduced and/or removed. By way of example, these valves 4 could also be holes that connect the thermal compartment 3 to the area outside the mattress 1.

In order to explain the mode of use of the invention, the operation of the thermal assembly in a preferred embodiment thereof is described below. In the situation where the thermal compartment 3 is empty and once the patient's need is determined, which refers to the provision of cold or heat, the chosen thermoactive substance is introduced through any of the valves 4 of the thermal compartment 3 by means of suitable injection elements, like in the case of a syringe adaptable to the valve designed for filling and/or emptying, as a manual pump. In the case that said thermoactive substance is a chemical mixture, said chemical mixture can be directly introduced into the thermal compartment 3 as a dissolution or suitable reagents can be introduced separately, such that the chemical mixture of the reagents is carried out inside the thermal compartment 3. Thus, the thermoactive substance generates or absorbs heat during a specific period of time to subsequently stabilise the temperature thereof, returning the thermoactive substance to room temperature.

When replacing said thermoactive substance contained in the thermal compartment 3 with the purpose of maintaining the transfer of heat or cold for a longer period of time or changing the substance used, the content of the thermal compartment 3 is emptied, injecting air through at least one of the valves 4 of the thermal compartment 3 by means of one of the injection elements mentioned previously and leaving at least one valve 4 of the thermal compartment 3 open to expel the thermoactive substance. Once the content of the thermal compartment 3 is emptied, the outlet valve is closed and it can be refilled with a desired substance through the inlet valve, restoring the thermal function of the thermal assembly. Therefore, the invention enables a reusable thermal assembly to be obtained.

By way of example, inducing hypothermia is especially suitable, for example, for patients with damaged tissues, traumas or who have suffered ischaemic diseases, among other conditions or accidents in desert areas. Furthermore, inducing hyperthermia is recommended for situations in which it is necessary to raise the patient's body temperature, for example, when said patient is suffering from hypothermia in situations of rescue at sea, in mountains or snow or in situations of isolation due to weather conditions, among other conditions. Thus, the present invention is designed to take on the different needs of the patient by simply introducing a suitable thermoactive substance into the thermal compartment 3, raising the patient's body temperature or lowering it when that patient is not able to make this change, such that the symptoms of certain ailments are treated from the start and during the transfer of the patient to the medical centre, without needing any electric device or connection to energy sources.

By way of example, and without restriction, the thermoactive substances mentioned in the present document can refer to several types of mixtures or compounds.

Firstly, they can refer to chemical mixtures of reagents that generate exothermic reactions, such as for example mixtures formed by:
a solution of magnesium sulphate and water,
a supersaturated solution of sodium acetate trihydrate and water.

Secondly, they can refer to chemical mixtures of reagents that generate endothermic reactions, such as for example
mixtures formed by ammonium in ratios greater than 95% with sodium carboxymethyl cellulose and with sodium chloride; or
chemical mixtures of nitrogenous salts and water.

In addition to the foregoing, they can also refer to compounds with thermal inertia that can transfer sensible heat for a certain period of time, such as in the case of water, or compounds that can transfer latent heat.

These chemical mixtures are biodegradable, meaning that they are not pollutants. For this reason, upon completing the function for which they were created, they can be discharged into the environment without a negative impact.

In embodiments of the invention, it is possible that, to achieve an exothermic reaction, the thermoactive substance used requires an element that disrupts the chemical balance, like in the case that said thermoactive substance is a hypersaturated solution of sodium acetate in water. In this case, the thermal compartment comprises a mechanical actuator therein that acts as an element for disrupting said chemical balance of the thermoactive substance. In a preferred embodiment, said mechanical actuator has blunt metal filings for nucleation located inside the thermal compartment or a small metal part that, upon being bent in contact with said hypersaturated solution of sodium acetate in water, disrupts the chemical balance and acts as a point of nucleation of the chemical solution, making it crystallise and quickly release latent heat.

Returning to the configuration of the mattress 1, it can be seen in Figure 2 that, in addition to the thermal function, it has a function of securing and protecting the patient during the transfer to the medical centre. To do so, the mattress 1 comprises, in addition to the thermal compartment 3, a vacuum compartment 5 arranged inside the mattress 1 and placed next to the thermal transfer surface 2 and the thermal compartment 3. Said vacuum compartment 5 comprises a gas therein, preferably air and a plurality of granulated components 6 of synthetic material. Moreover, the vacuum compartment 5 has means for introducing and removing the gas from the inside, also present in the transportation means for emergencies, and which will be adapted to the vacuum valve 7 of the vacuum compartment. The vacuum valve was also shown in Figure 1.

In a preferred embodiment of the invention, the granulated components 6 of synthetic material are balls of polyethylene. Likewise, the means for introducing and removing gases from the inside of the vacuum compartment 5 comprise at least one vacuum valve 7.

In another preferred embodiment of the invention, the material that makes up the vacuum compartment 5 is manufactured from polyvinyl chloride (PVC), although in other embodiments it can be polyurethane, such that it provides said vacuum compartment 5 with a certain rigidity to the structure during the removal of the gas from the inside of said vacuum compartment 5.

To help understand the nature of the present invention, the mode of use of this configuration of the invention comprising the vacuum compartment 5 is described below. By positioning a patient on the thermal transfer surface 2, the very weight of the patient causes the granulated components 6 to be shaped to the morphology of the body. Thus, once the air has been removed from the inside of the vacuum compartment 5 through the one or more vacuum valves 7 by means of, for example, a vacuum pump, the mattress 1 remains rigidly adapted to the patient thanks to the action of the vacuum compartment 5 due to the compaction of the granulated components 6.

The vacuum function is especially suitable for transporting patients that have suffered severe traumas and need to be immobilised. Thus, a device is obtained that is capable of immobilising the injuries of the patient and absorbing most of the vibrations during the transportation of said patient to a medical centre, at the same time that it enables a thermal treatment to be applied to the patient, providing or absorbing heat.

Furthermore, the mattress has gripping elements. In a preferred embodiment of the invention, said gripping elements are made up of three handles 8, preferably distributed along the perimeter of the surface of the mattress 1, such that it enables the manual transportation of the patient, for example, in an emergency situation. Thus, a mattress is obtained that in the thermal assembly thereof is portable and suitable for transporting a patient.

Likewise, the invention has elements for securing the patient to the mattress 1, as shown in Figures 1 and 2. Said securing elements comprise, in a preferred embodiment, a strap 9 on each one of the handles 8 distributed along the thermal transfer surface 2, such that it can secure the patient to the mattress 1 and provide greater stability and less risk of falling to said patient during transportation. These straps can be moved by the handle to which they are fastened in order to adapt them to the measurements of the patient and offer better adaptability to the assembly not offered until now.

In another preferred embodiment of the invention, both the content of the vacuum compartment 5 and the content of the thermal compartment 3 are materials permeable to X-rays, CT or computerised tomography, and electromagnetic radiation produced by the nuclear magnetic resonance or NMR technique. Due to this, the invention enables these diagnostic tests to be carried out quickly once the patient has been transferred to the medical centre, without needing to transfer said patient to another support device, an action that is generally complex in patients in an emergency and that maintains the transfer of heat if necessary and keeps the patient immobilised during said tests.

Another object of the present invention relates to a kit for the thermal treatment of a patient comprising a thermal assembly according to the different embodiments described previously, a thermoactive substance and elements for injecting the thermoactive element inside the thermal compartment.

In a preferred embodiment of the invention, the injection elements of the kit comprise some of the following elements: a syringe and a manual pump for the thermal compartment, and a manual pump, electric pump and/or a secretion aspirator in the case of the vacuum compartment.

Thus, an invention is obtained that enables the early thermal treatment of patients, generally in emergency situations, suitable for being used during the transportation of patients in emergency situations, by means of a reusable and portable invention that can also be suitable for immobilising and absorbing vibrations and impacts due to the functionality of the vacuum compartment 5.

## Claims

1. A thermal mattress (1) for body support, suitable for treating a patient during their transportation in emergency situations, **characterised in that** it comprises
a thermal transfer surface (2) with an outer face adapted to receive the body of a patient and an inner face;
a thermal compartment (3) arranged inside the mattress in contact with the inner face of the thermal transfer surface, the thermal compartment (3) being adapted to house a thermoactive substance;
transfer means (4) for transferring the thermoactive substance between the thermal compartment (3) and an area outside the mattress (1).

2. The mattress (1) according to the preceding claim, wherein the thermal transfer surface is rough and slip resistant.

3. The mattress (1) according to any of the preceding claims, wherein the thermal compartment (3) is distributed longitudinally through the inside of the mattress and is welded to the inner face of the thermal transfer surface.

4. The mattress (1) according to any of the preceding claims, which further comprises a vacuum compartment (5), the thermal compartment (3) being located between said vacuum compartment (5) and the thermal transfer surface (2), and wherein said vacuum compartment (5) comprises:
a vacuum valve (7) for injecting or removing a gas from the inside of the vacuum compartment (5); and
a plurality of moulding granulated components (6) inside the vacuum compartment (5).

5. The mattress (1) according to the preceding claim, wherein the thermal compartment (3) and/or the vacuum compartment (5) are impermeable to liquids and/or gases.

6. The mattress (1) according to any of claims 4 or 5, wherein the granulated components (6) of the vacuum compartment (5) are spheres.

7. The mattress (1) according to the preceding claim, wherein the spheres (6) of the vacuum compartment (5) comprise polyethylene.

8. The mattress (1) according to any of claims 4 to 7, wherein the material of the vacuum compartment (5) comprises polyvinyl chloride or polyurethane.

9. The mattress (1) according to any of the preceding claims, wherein the transfer means for transferring the thermoactive substance comprise at least two valves (4) located in the thermal compartment (3).

10. The mattress (1) according to any of the preceding claims, which further comprises temperature sensors arranged on the thermal transfer surface (2) of the mattress (1).

11. The mattress (1) according to any of the preceding claims, which further comprises one or more handles (8) located on the sides of the mattress (1).

12. The mattress (1) according to claim 11, wherein the mattress (1) comprises three or more straps and each strap (9) has an end joined in a sliding manner to a handle (8), such that by sliding the end along the handle, the position of the straps (9) is adjusted.

13. An assembly according to any of the preceding claims, wherein the thermal compartment (3) comprises a mechanical actuator therein.

14. A kit for the thermal treatment and body support of a patient comprising: a mattress (1), according to any of the preceding claims, at least one thermoactive substance, at least one injection element for injecting the thermoactive element inside the thermal compartment (3) through the transfer means.

15. The kit according to the preceding claim, wherein the injection means comprise a syringe with dimensions suited for the transfer means, and wherein the kit additionally comprises a manual pump, an electric pump and/or a secretion aspirator suitable for filling or emptying the vacuum compartment.
